# EUROPEAN PATENT APPLICATION

(11) **EP 3 238 721 A1**
(43) Date of publication of application: **01.11.2017**
(21) Application number: 15873246.1
(22) Date of filing: 25.12.2015
(51) Int. Cl.: A61K 31/444, A61K 36/81, A61K 45/00, A61P 7/02, A61P 43/00

(54) **MEDICAL COMPOSITION FOR PROMOTING FIBRINOLYSIS**

(30) Priority: 26.12.2014 JP 2014263765
(71) Applicant: Daiichi Sankyo Company, Limited, Tokyo 103-8426 (JP)
(72) Inventor: FURUGORI, Taketoshi, Tokyo 140-8710 (JP); MORISHIMA, Yoshiyuki, Tokyo 140-8710 (JP)
(74) Representative: Fairbairn, Angus Chisholm
(86) International application number: PCT/JP2015/086178
(87) International publication number: WO 2016/104678

(57) **Abstract**

It is intended to provide a novel pharmaceutical composition that can promote fibrinolysis. The present invention provides a pharmaceutical composition for the promotion of fibrinolysis, comprising edoxaban or a pharmaceutically acceptable salt thereof, or a hydrate of the compound or the salt. The present invention further provides a pharmaceutical composition for the promotion of fibrinolysis, comprising edoxaban or a pharmaceutically acceptable salt thereof, or a hydrate of the compound or the salt and further comprising a TAFIa inhibitor.

## Description

### Technical Field

The present invention relates to a pharmaceutical composition for the promotion of fibrinolysis, comprising edoxaban or a pharmaceutically acceptable salt thereof, or a hydrate of the compound or the salt, a method for promoting fibrinolysis using edoxaban or a pharmaceutically acceptable salt thereof, or a hydrate of the compound or the salt, and combined use or a combination drug of edoxaban or a pharmaceutically acceptable salt thereof, or a hydrate of the compound or the salt with an agent having a TAFIa inhibitor for the promotion of fibrinolysis.

### Background Art

Edoxaban tosylate hydrate competitively and selectively inhibits activated blood coagulation factor X (hereinafter, referred to as "FXa") in mammals such as humans.

Pharmaceutical compositions containing edoxaban tosylate hydrate have indications and usage, such as reduction in the risks of stroke and systemic embolism in non-valvular atrial fibrillation patients, and treatment of deep vein thrombosis and pulmonary embolism (see e.g., Non-Patent Literatures 1 and 2).

Urokinase, streptokinase, tPA, and the like are used for lysing thrombus (see e.g., Non-Patent Literature 3).

### Citation List

### Non-Patent Literature

Non-Patent Literature 1: Blood (ASH Annual Meeting Abstracts) 2012 120: Abstract 4697
Non-Patent Literature 2: SAVAYSA Label
Non-Patent Literature 3: Journal of vascular surgery, Vol. 22, No. 5, 1995, p. 593-597

### Summary of Invention

### Technical Problem

An object of the present invention is to provide a method for promoting fibrinolysis using an FXa inhibitor such as edoxaban or using an FXa inhibitor such as edoxaban and a TAFIa inhibitor.

### Solution to Problem

The present invention provides:
(1) a pharmaceutical composition for the promotion of fibrinolysis, comprising edoxaban or a pharmaceutically acceptable salt thereof, or a hydrate of the compound or the salt;
(2) the pharmaceutical composition according to (1), further comprising a TAFIa inhibitor;
(3) the pharmaceutical composition according to (2), wherein the TAFIa inhibitor is a carboxypeptidase inhibitor from potato tuber;
(4) use of edoxaban or a pharmaceutically acceptable salt thereof, or a hydrate of the compound or the salt for producing a pharmaceutical composition for the promotion of fibrinolysis;
(5) the use according to (4), wherein a TAFIa inhibitor is further used;
(6) the use according to (5), wherein the TAFIa inhibitor is a carboxypeptidase inhibitor from potato tuber;
(7) a method for promoting fibrinolysis, comprising administering edoxaban or a pharmaceutically acceptable salt thereof, or a hydrate of the compound or the salt to a mammal;
(8) the method according to (7), comprising
   administering a TAFIa inhibitor to the mammal at the same time as or separately from the edoxaban or the pharmaceutically acceptable salt thereof, or the hydrate of the compound or the salt;
(9) the method according to (7) or (8), wherein the mammal is a human; and
(10) an agent promoting fibrinolysis, comprising edoxaban or a pharmaceutically acceptable salt thereof, or a hydrate of the compound or the salt in combination with a TAFIa inhibitor.

### Advantageous Effects of Invention

The present invention exerts an effect in that a novel pharmaceutical composition that can promote fibrinolysis can be provided.

### Brief Description of Drawings

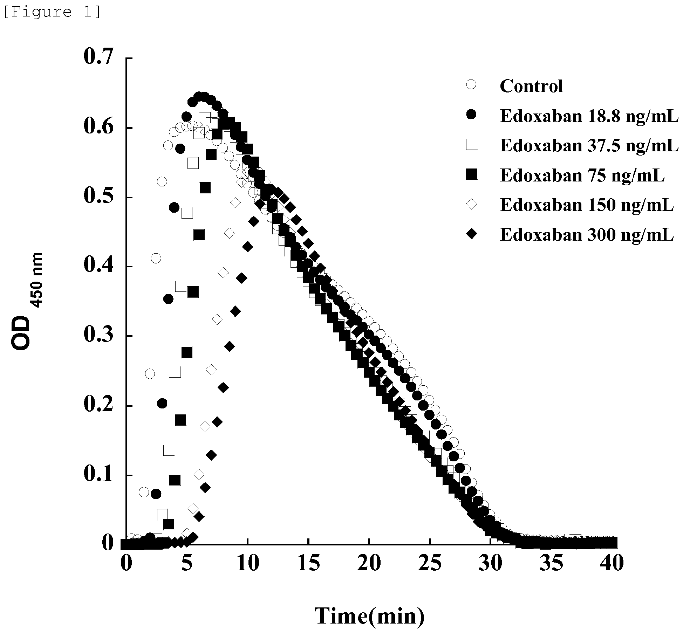

[Figure 1] Figure 1 is a diagram showing the influence of edoxaban on fibrinolytic phenomenon induced by tPA in human plasma.

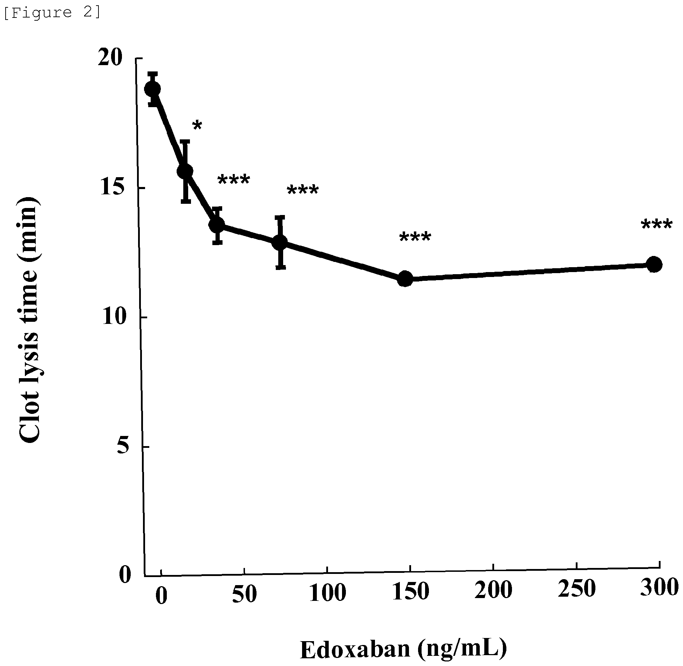

[Figure 2] Figure 2 is a diagram showing the influence of edoxaban on clot lysis time induced by tPA in human plasma. In the drawing, significant difference from control by Dunnett's multiple comparison test is indicated by * (P < 0.05) and *** (P < 0.001).

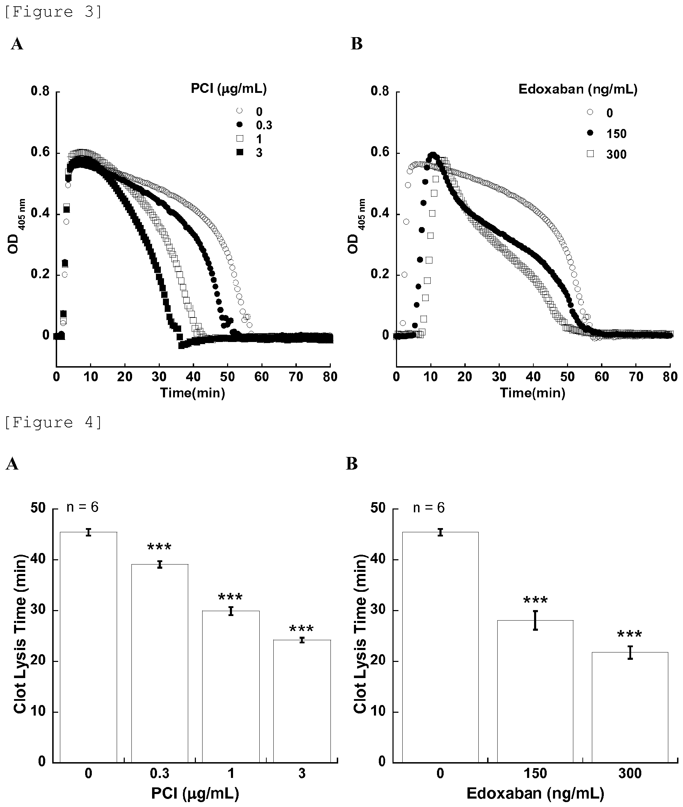

[Figure 3] Figure 3 is a diagram showing the influence of edoxaban (Figure 3A) or PCI (Figure 3B) on fibrinolytic phenomenon induced by tPA in human plasma.
[Figure 4] Figure 4 is a diagram showing the influence of edoxaban (Figure 4B) or PCI (Figure 4A) on clot lysis time induced by tPA in human plasma. In the drawing, significant difference from control by paired t-test with Bonferroni correction is indicated by *** (P < 0.001). In this context, the control refers to a group involving neither PCI nor edoxaban.

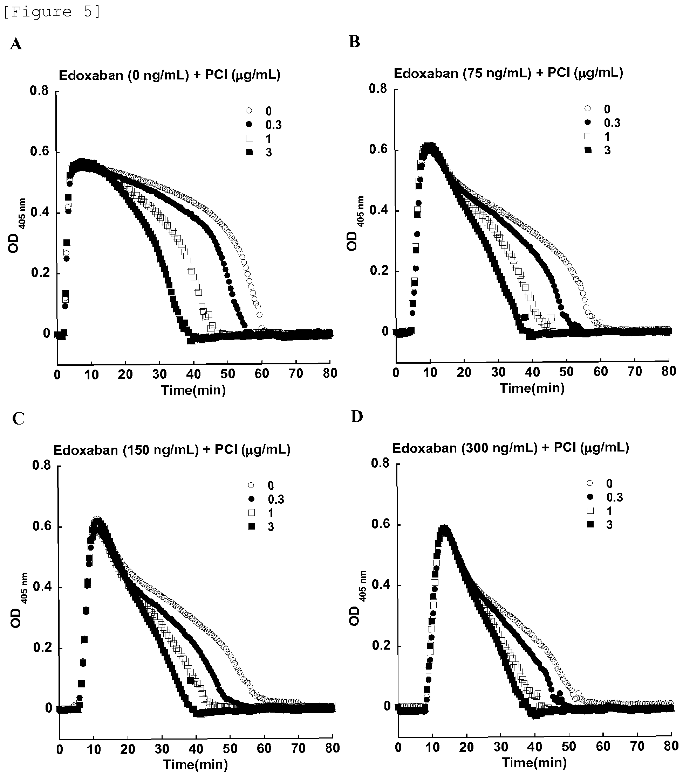

[Figure 5] Figure 5 is a diagram showing the influence of edoxaban, PCI, and combinations thereof on fibrinolytic phenomenon induced by tPA in human plasma. Figure 5A shows the influence of PCI, Figure 5B shows the influence of edoxaban, Figure 5C shows the influence of the combined use of 150 ng/mL edoxaban and PCI, and Figure 5D shows the influence of the combined use of 300 ng/mL edoxaban and PCI.

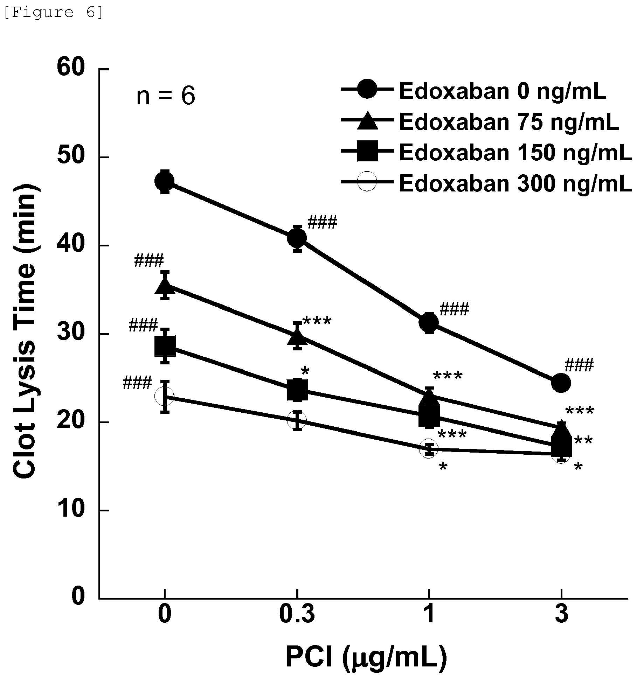

[Figure 6] Figure 6 is a diagram showing the influence of edoxaban, PCI, and combinations thereof on clot lysis time induced by tPA in human plasma. In the drawing, significant difference from control by paired t-test with Bonferroni correction is indicated by ### (P < 0.001). In this context, the control refers to a group involving neither PCI nor edoxaban. In the drawing, significant difference from 75, 150, or 300 ng/mL edoxaban by paired t-test with Bonferroni correction is indicated by * (P < 0.05), ** (P < 0.01), and *** (P < 0.001).

### Description of Embodiments

In the present specification, the term "fibrinolysis" refers to lysis of thrombus.

In the present specification, the term "edoxaban" means N-(5-chloropyridin-2-yl)-N'-[(1S,2R,4S)-4-(dimethylcarbamoyl)-2-(5-methyl-4,5,6,7-tetrahydro[1,3]thiazolo[5,4-c]pyridine-2-carboxamido)cyclohexyl]oxamide represented by the following formula: 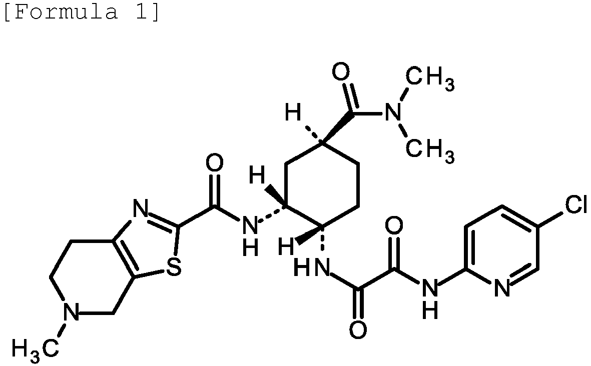 unless otherwise specified.

Examples of salts of edoxaban include hydrochloride, sulfate, hydrobromide, citrate, hydroiodide, phosphate, nitrate, benzoate, methanesulfonate, benzenesulfonate, 2-hydroxyethanesulfonate, tosylate, acetate, propanoate, oxalate, malonate, succinate, glutarate, adipate, tartrate, maleate, fumarate, malate and mandelate. The salt of edoxaban is preferably hydrochloride, tartrate or tosylate, more preferably tosylate.

The edoxaban or the pharmaceutically acceptable salt thereof, or the hydrate of the compound or the salt is preferably edoxaban tosylate hydrate represented by the following formula:

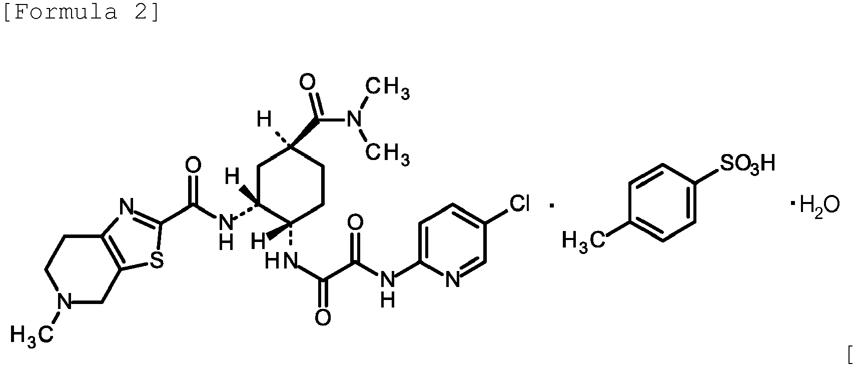

A commercially available product can be used as the edoxaban or the pharmaceutically acceptable salt thereof, or the hydrate of the compound or the salt.

In the present specification, the term "TAFI" refers to a thrombin-activatable fibrinolysis inhibitor. In the present specification, the term "TAFIa" refers to activated TAFI. In the present specification, the term "TAFIa inhibitor" refers to an agent inhibiting TAFIa.

Examples of the TAFIa inhibitor used in the present invention include a carboxypeptidase inhibitor from potato tuber (hereinafter, also referred to as "PCI"), 5-amino-2-[(1-Cyclohexyl-1H-imidazol-4-yl)methyl]valeric acid, 5-amino-2-{[1-(4-methylcyclohexyl)-1H-imidazol-4-yl]methyl}valeric acid, 5-amino-2-{[1-(4-ethylcyclohexyl)-1H-imidazol-4-yl]methyl}valeric acid, 5-amino-2-{[1-(3-ethylcyclobutyl)-1H-imidazol-4-yl]methyl}valeric acid, 5-amino-2-{[1-(3-methylcyclobutyl)-1H-imidazol-4-yl]methyl}valeric acid, 5-amino-2-({1-[(1R,3s,5S)-bicyclo[3.1.0]hex-3-yl]-1H-imidazol-4-yl}methyl)valeric acid, 5-amino-2-{[1-(4-hydroxycyclohexyl)-1H-imidazol-4-yl]methyl}valeric acid, 5-amino-2-{[1-(4-hydroxy-4-methylcyclohexyl)-1H-imidazol-4-yl]methyl}valeric acid, 5-amino-2-{[1-(3-methylcyclohexyl)-1H-imidazol-4-yl]methyl}valeric acid, 5-amino-2-[(1-cycloheptyl-1H-imidazol-4-yl)methyl]valeric acid, 5-amino-2-({1-[exo-bicyclo[2.2.1]hept-2-yl]-1H-imidazol-4-yl}methyl)valeric acid, 5-amino-2-({1-[endobicyclo[2.2.1]hept-2-yl]-1H-imidazol-4-yl}methyl)valeric acid, 2-[(1-adamantan-2-yl-1H-imidazol-4-yl)methyl]-5-aminovaleric acid, 5-amino-2-{[1-(4-phenoxycyclohexyl)-1H-imidazol-4-yl]methyl}valeric acid, benzyl 5-amino-2-{[1-(4-methylcyclohexyl)-1H-imidazol-4-yl]methyl}valerate, 2-{[1-(4-methylcyclohexyl)-1H-imidazol-4-yl]methyl}-5-(L-phenylalanylamino)valeric acid, 2-{[1-(4-methylcyclohexyl)-1H-imidazol-4-yl]methyl}-5-(L-norleucylamino)valeric acid, 2-{[1-(4-methylcyclohexyl)-1H-imidazol-4-yl]methyl}-5-({[(5-methyl-2-oxo-1,3-dioxol-4-yl)methoxy]carbonyl}amino)valeric acid, 5-({[1-(isobutyryloxy)ethoxy]carbonyl}amino)-2-{[1-(4-methylcyclohexyl)-1H-imidazol-4-yl]methyl}valeric acid, 1-[(isopropoxycarbonyl)oxy]ethyl 5-({[1-(isobutyryloxy)ethoxy]carbonyl}amino)-2-{[1-(4-methylcyclohexyl)-1lH-imidazol-4-yl]methyl}valerate, 5-({[1-(2,2-dimethylpropanoyloxy)ethoxy]carbonyl}amino)-2-{[1-(4-methylcyclohexyl)-1H-imidazol-4-yl]methyl}valeric acid, 5-[({1-[(cyclohexylcarbonyl)oxy]ethoxy}carbonyl)amino]-2-{[1-(4-methylcyclohexyl)-1H-imidazol-4-yl]methyl}valeric acid, 2-(2-aminoethoxy)-3-[1-(4-methylcyclohexyl)-1H-imidazol-4-yl]propionic acid, 2-[(1R)-2-amino-1-methylethoxy]-3-[1-(4-methylcyclohexyl)-1H-imidazol-4-yl]propionic acid, 2-[(3S)-3-aminopyrrolidin-1-yl]-3-[1-(4-methylcyclohexyl)-1H-imidazol-4-yl]propionic acid, (2S)-2-{[1-(trans-4-methylcyclohexyl)-1H-imidazol-4-yl]methyl}-5-({[(5-methyl-2-oxo-1,3-dioxol-4-yl)methoxy]carbonyl}amino)valeric acid, (2S)-5-({[1-(isobutyryloxy)ethoxy]carbonyl}amino)-2-{[1-(trans-4-methylcyclohexyl)-1H-imidazol-4-yl]methyl}valeric acid, 1-[(isopropoxycarbonyl)oxy]ethyl (2S)-5-({[1-(isobutyryloxy)ethoxy]carbonyl}amino)-2-{[1-(trans-4-methylcyclohexyl)-1H-imidazol-4-yl]methyl}valerate, (2S)-5-({[1-(2,2-dimethylpropanoyloxy)ethoxy]carbonyl}amino)-2-{[1-(trans-4-methylcyclohexyl)-1H-imidazol-4-yl]methyl}valeric acid, (2S)-5-[({1-[(cyclohexylcarbonyl)oxy]ethoxy}carbonyl)amino]-2-{[1-(trans-4-methylcyclohexyl)-1H-imidazol-4-yl]methyl}valeric acid, (2S)-5-{[(1-acetoxyethoxy)carbonyl]amino}-2-{[1-(trans-4-methylcyclohexyl)-1H-imidazol-4-yl]methyl}valeric acid, (2S)-2-{[1-(trans-4-methylcyclohexyl)-1H-imidazol-4-yl]methyl}-5-[({[(2-methylpropanoyl)oxy]methoxy}carbonyl)amino]valeric acid, (2S)-5-[({[(2,2-dimethylpropanoyl)oxy]methyloxy}carbonyl)amino]-2-{[1-(trans-4-methylcyclohexyl)-1H-imidazol-4-yl]methyl}valeric acid, (2S)-5-[({[(cyclohexylcarbonyl)oxy]methoxy}carbonyl)amino]-2-{[1-(trans-4-methylcyclohexyl)-1H-imidazol-4-yl]methyl}valeric acid, (2S)-5-({[(acetyloxy)methoxy]carbonyl}amino)-2-{[1-(trans-4-methylcyclohexyl)-1H-imidazol-4-yl]methyl}valeric acid, (2S)-5-({[(1R)-1-(isobutyryloxy)ethoxy]carbonyl}amino)-2-{[1-(trans-4-methylcyclohexyl)-1H-imidazol-4-yl]methyl}valeric acid, 2-(3-aminopropyl)-1-(1-phenyl-1H-imidazol-4-yl)cyclopropanecarboxylic acid, 2-(3-aminopropyl)-1-[1-(3,3-dimethylbutyl)-1H-imidazol-4-yl]cyclopropanecarboxylic acid, 2-(3-aminopropyl)-1-[1-(trans-4-methylcyclohexyl)-1H-imidazol-4-yl]cyclopropanecarboxylic acid, 2-(3-aminopropyl)-1-(1-pyridin-2-yl-1H-imidazol-4-yl)cyclopropanecarboxylic acid, 2-(3-amino-2-methylpropyl)-1-(1-phenyl-1H-imidazol-4-yl)cyclopropanecarboxylic acid, 2-(3-aminopropyl)-1-[1-(5-methylpyridin-2-yl)-1H-imidazol-4-yl]cyclopropanecarboxylic acid, 2-[(2-aminomethyl)butyl]-1-(1-phenyl-1H-imidazol-4-yl)cyclopropanecarboxylic acid, 2-[(2R)-3-amino-2-methylpropyl]-1-(1-pyridin-2-yl-1H-imidazol-4-yl)cyclopropanecarboxylic acid, 2-[(2R)-3-amino-2-methylpropyl]-1-[1-(5-methylpyridin-2-yl)-1H-imidazol-4-yl]cyclopropanecarboxylic acid, 2-[(2R)-2-(aminomethyl)butyl]-1-(1-pyridin-2-yl-1H-imidazol-4-yl)cyclopropanecarboxylic acid, (1R,2S)-2-(3-aminopropyl)-1-(1-phenyl-1H-imidazol-4-yl)cyclopropanecarboxylic acid, (1R,2S)-2-(3-aminopropyl)-1-(1-pyridin-2-yl-1H-imidazol-4-yl)cyclopropanecarboxylic acid, (1R,2S)-2-[(2R)-3-amino-2-methylpropyl]-1-(1-pyridin-2-yl-1H-imidazol-4-yl)cyclopropanecarboxylic acid, (1R,2S)-2-[(2R)-3-amino-2-methylpropyl]-1-[1-(5-methylpyridin-2-yl)-1H-imidazol-4-yl]cyclopropanecarboxylic acid, (1R,2S)-2-[(2R)-2-(aminomethyl)butyl]-1-(1-pyridin-2-yl-1H-imidazol-4-yl)cyclopropanecarboxylic acid, SAR-126119, SAR-104772, UK-396082, BX-528, AZD-9684, and EF-6265/MN-462.

In the present specification, the phrase "comprising edoxaban or a pharmaceutically acceptable salt thereof, or a hydrate of the compound or the salt in combination with an agent having a TAFIa inhibitor" refers to a form in which a preparation containing the edoxaban or the pharmaceutically acceptable salt thereof, or the hydrate of the compound or the salt is administered at the same time with or separately from a preparation containing the TAFIa inhibitor, or a form in which a preparation containing both of the edoxaban or the pharmaceutically acceptable salt thereof and the TAFIa inhibitor (hereinafter, referred to as a "combination drug") is administered.

In the present specification, administration "at the same time" refers to administration at substantially the same time.

In the present specification, "separate" administration refers to separate administration at different times. For example, the TAFIa inhibitor is first administered, and subsequently, the edoxaban or the pharmaceutically acceptable salt thereof, or the hydrate of the compound or the salt is administered after elapse of a predetermined time, or vice versa.

In the case of administering the pharmaceutical composition or the combination drug of the present invention to a mammal (e.g., a human, a horse, cattle or a pig, preferably a human), the administration can be systemic or local and oral or parenteral.

The pharmaceutical composition or the combination drug of the present invention can be prepared according to various formulation methods usually used by selecting an appropriate form according to the administration method.

Examples of oral dosage forms of the pharmaceutical composition or the combination drug include tablets, powders, granules, capsules, suspensions, emulsions, syrups and elixirs. The pharmaceutical composition in such a form can be produced according to a routine method by selecting an additive usually used such as an excipient, a binder, a disintegrant, a lubricant, a swelling agent, a swelling aid, a coating agent, a plasticizer, a stabilizer, an antiseptic, an antioxidant, a colorant, a solubilizer, a suspending agent, an emulsifier, a sweetener, a preservative, a buffer, a diluent or a wetting agent according to need.

Examples of parenteral dosage forms of the pharmaceutical composition or the combination drug include injections, ointments, gels, creams, poultices, patches, aerosolized agents, inhalants, sprays, eye drops, nasal drops and suppositories. The pharmaceutical composition in such a form can be produced according to a routine method by selecting an additive usually used such as a stabilizer, an antiseptic, a solubilizer, a humectant, a preservative, an antioxidant, a flavor, a gelling agent, a neutralizing agent, a buffer, a tonicity agent, a surfactant, a colorant, a buffering agent, a thickener, a wetting agent, a filler, an absorption promoter, a suspending agent or a binder according to need.

The dose of the edoxaban or the pharmaceutically acceptable salt thereof, or the hydrate of the compound or the salt contained in the pharmaceutical composition of the present invention differs depending on symptoms, age, body mass, etc. For oral administration, the edoxaban or the pharmaceutically acceptable salt thereof, or the hydrate of the compound or the salt is administered once to several times a day at a dose of 1 to 200 mg, preferably 5 to 100 mg, more preferably 15 to 60 mg, in terms of the amount of edoxaban per dose in an adult. When the edoxaban or the pharmaceutically acceptable salt thereof, or the hydrate of the compound or the salt is used in combination with a TAFIa inhibitor, an appropriate decrease in its dose is also taken into consideration in light of the risk of bleeding.

The pharmaceutical composition or the combination drug thus obtained can be used for promoting fibrinolysis. Thus, the pharmaceutical composition or the combination drug can be used for, for example, the suppression of occurrence of venous thromboembolism (deep venous thrombosis and pulmonary thromboembolism), the treatment and the suppression of recurrence of venous thromboembolism, the suppression of occurrence of ischemic stroke and systemic embolism in non-valvular atrial fibrillation patients, the suppression of occurrence of postthrombotic syndrome, the suppression of occurrence of chronic thromboembolic pulmonary hypertension, the lysis of arterial thrombus in ischemic cerebral infarction and acute coronary syndrome, the suppression of recurrence of ischemic cerebral infarction and acute coronary syndrome, and the treatment and the suppression of recurrence of peripheral arterial disease.

Next, the present invention will be described in detail with reference to the Examples. However, the present invention is not intended to be limited by these in any way.

### Examples

### (Example 1) Fibrinolysis promoting effect of edoxaban

### (1) Preparation of test substance solution

Edoxaban was dissolved in dimethyl sulfoxide (hereinafter, also referred to as "DMSO") (Wako Pure Chemical Industries, Ltd.) to prepare a 10 mM solution, which was then stored at -30°C. Before use, the frozen product was thawed and diluted with DMSO and saline (Otsuka Pharmaceutical Factory, Inc.) (DMSO concentration in test substance solution: 0.96%, final DMSO concentration in reaction system: 0.08%).

### (2) Preparation of reagent

Hepes buffer: Hepes (Sigma-Aldrich, Co. LLC.) and NaCl (Nacalai Tesque Inc.) were dissolved at 20 mM and 140 mM, respectively, in distilled water, and the solution was adjusted to pH 7.4 using a 1 mol/L sodium hydroxide solution (Nacalai Tesque Inc.). The buffer was stored at 4°C, and BSA (final concentration: 0.01%) (Sigma-Aldrich, Co. LLC.) was added thereto in use.

tPA (ACTIVACIN for Injection 6 million, Kyowa Hakko Kirin Co., Ltd.) solution: The solution was prepared at 1.034 mg/mL (600,000 U/mL) with injectable water included in the product, and stored at -80°C. Before use, the frozen product was thawed and diluted with Hepes buffer for use.

PPP reagent (Thrombinoscope BV): 1 vial of freeze dried product was dissolved in 1 mL of distilled water (Otsuka Pharmaceutical Factory, Inc.), and this solution was diluted 2-fold with phospholipid (liposome freeze dried product, E-PC/E-PE/B-PS = 60/20/20) solution and used (final concentration: 2.5 pM tissue factor / 4 µM phospholipids).

### (3) Preparation of human plasma sample

26 µL of a 10.34 µg/mL tPA solution was added to 1473 µL of human plasma (FACT, George King Bio-Medical, Inc.) to prepare a human plasma sample having a concentration of 180 ng/mL in plasma.

### (4) Fibrinolysis assay

70 µL of the human plasma sample (containing tPA), 10 µL each of varying concentrations of the edoxaban solution, and 20 µL of the PPP reagent were added to each well of a 96-well plate (Sumilon Proteosave SS 96F plate, MS-8296F). After preincubation at 37°C for approximately 10 minutes, the reaction was started by the addition of 20 µL/well of a Fluo buffer (Hepes buffer containing 100 mM CaCl₂) (Thrombinoscope BV). Absorbance at 405 nm was measured every 30 seconds using SpectraMAX 384 Plus (Molecular Devices, LLC.) prewarmed to 37°C. The time for the absorbance to return to 1/2 of the peak from the time to reach 1/2 of the peak was calculated using SoftMax Pro 5.4.1 (Molecular Devices, LLC.) and used as clot lysis time. A group using a 0.96% DMSO solution instead of edoxaban was used as control group.

### (5) Statistical analysis

Two experiments were conducted on each of 3 lots of human plasma (n = 6). The basic statistics (mean, standard deviation, and standard error) of the clot lysis time were calculated, and the results were indicated by mean ± standard error (SEM). The control group and the edoxaban group were compared by Dunnett's test, and concentration dependence was evaluated by Spearman's rank correlation coefficient test (significance level for both of the tests: two-sided 5%). The calculated P value was rounded off and indicated by three decimal places. Microsoft Excel 2010 (Microsoft Corporation) and SAS System Release 9.2 (SAS Institute Inc.) were used in the analysis. When the clot lysis time of the edoxaban group exhibited a significantly lower value than that of the control group, a fibrinolysis promoting effect was determined.

### (6) Results

Changes in absorbance are shown in Figure 1. The clot lysis time is shown in Figure 2. The statistical analysis results are shown in Table 1. Edoxaban significantly shortened the clot lysis time in a concentration-dependent manner in the concentration range of 18.8 ng/mL to 300 ng/mL and exhibited a fibrinolysis promoting effect.

**[Table 1]**

| No. | Comparison group | Method of analysis | *P* value |
|---|---|---|---|
| 1 | Control vs Edoxaban 18.8 ng/mL | Dunnett multiple comparison test | 0.015 |
| | Control vs Edoxaban 37.5 ng/mL | | <0.001 |
| | Control vs Edoxaban 75 ng/mL | | <0.001 |
| | Control vs Edoxaban 150 ng/mL | | <0.001 |
| | Control vs Edoxaban 300 ng/mL | | <0.001 |
| 2 | Control, Edoxaban 18.8 ng/mL, Edoxaban 37.5 ng/mL, Edoxaban 75 ng/mL, Edoxaban 150 ng/mL, Edoxaban 300 ng/mL | Spearman's rank correlation | <0.001 |

### (Example 2) Fibrinolysis promoting effect of combined use of edoxaban and TAFI inhibitor

### (1) Preparation of test substance solution

Edoxaban was dissolved in DMSO to prepare a 10 mM solution, which was then stored at -30°C. Before use, the frozen product was thawed and diluted with DMSO and saline (DMSO concentration in test substance solution: 1.92%).

PCI (Sigma-Aldrich, Co. LLC.) was dissolved in Hepes buffer to prepare a 4.8 mg/mL solution, which was then stored at -30°C. Before use, the frozen product was thawed and diluted with Hepes buffer.

### (2) Preparation of reagent

Hepes buffer, tPA solution, and PPP reagent were prepared in the same way as in Example 1(2).

Thrombomodulin (hereinafter, also referred to as "TM") (Recomodulin for Intravenous Injection 12800, Asahi Kasei Pharma Corporation) was adjusted to 15.6 µM (1 mg/mL) with saline and stored at -30°C. Before use, the frozen product was thawed and diluted with saline for use.

### (3) Preparation of human plasma sample

47 µL each of a 10.8 µg/mL tPA solution and a 6 nM TM solution was added to 2707 µL of human plasma (FACT) to prepare a human plasma sample (concentration in plasma: tPA: 180 ng/mL, TM: 0.1 nM).

### (4) Fibrinolysis assay

70 µL of the human plasma sample (containing tPA and TM), 5 µL each of varying concentrations of the edoxaban solution, 5 µL of PCI, and 20 µL of the PPP reagent were added to each well of a 96-well plate (Sumilon Proteosave SS 96F plate, MS-8296F). After preincubation at 37°C for approximately 10 minutes, the reaction was started by the addition of 20 µL/well of a Fluo buffer (Hepes buffer containing 100 mM CaCl₂). Absorbance at 405 nm was measured every 30 seconds using SpectraMAX 384 Plus prewarmed to 37°C. The time for the absorbance to decrease to 1/2 of the peak from the time to reach 1/2 of the peak was calculated using SoftMax Pro 5.4.1 and used as clot lysis time. A group using a 1.92% DMSO solution and saline instead of edoxaban and PCI was used as control group.

### (5) Statistical analysis

Two experiments were conducted on each of 3 lots of human plasma (n = 6). The basic statistics (mean, standard deviation, and standard error) of the clot lysis time were calculated, and the results were indicated by mean ± standard error (SEM). The comparison between the control group and the edoxaban-alone group or the PCI-alone group and the comparison between the edoxaban-alone group or the PCI-alone group and the edoxaban + PCI combined use group were carried out by paired t-test (Bonferroni correction). Concentration dependence was evaluated by Spearman's rank correlation coefficient hypothesis test. The significance level of the tests was set to two-sided 5%. The calculated P value was rounded off and indicated by three decimal places. Microsoft Excel 2010 and SAS System Release 9.2 were used in the statistical analysis.

### (6) Results

Changes in absorbance caused by edoxaban and PCI each used alone are shown in Figure 3. The clot lysis times are shown in Figure 4. The statistical analysis results are shown in Table 2. Edoxaban significantly shortened the clot lysis time in a concentration-dependent manner at concentrations of 150 and 300 ng/mL. PCI significantly shortened the clot lysis time in a concentration-dependent manner at concentrations of 0.3 to 3 µg/mL.

Next, changes in absorbance caused by the combined use of edoxaban and PCI are shown in Figure 5. The clot lysis times are shown in Figure 6. The statistical analysis results are shown in Tables 3 and 4. Edoxaban and PCI when used in combination significantly shortened the clot lysis time as compared with their respective alone groups (except for the comparison between 300 ng/mL edoxaban used alone and 300 ng/mL edoxaban + 0.3 µg/mL PCI used in combination), demonstrating that the combined use of these inhibitors is effective.

**[Table 2]**

| No. | Comparison group | Method of analysis | *P* value |
|---|---|---|---|
| 1 | Control vs PCI 0.3 µg/mL | Paired t-test (Bonferroni correction)# | <0.001 |
| | Control vs PCI 1 µg/mL | | <0.001 |
| | Control vs PCI 3 µg/mL | | <0.001 |
| 2 | Control vs Edoxaban 150 ng/mL | Paired t-test (Bonferroni correction)* | <0.001 |
| | Control vs Edoxaban 300 ng/mL | | <0.001 |
| 3 | Control, PCI 0.3 µg/mL, PCI 1 µg/mL, PCI 3 µg/mL | Spearman's rank correlation coefficient hypothesis test | <0.001 |
| 4 | Control, Edoxaban 150 ng/mL, Edoxaban 300 ng/mL | Spearman's rank correlation coefficient hypothesis test | <0.001 |

| | | | |
|---|---|---|---|
| #: The control group and the PCI-alone group were compared by the paired t-test (Bonferroni correction). The adjusted P value was calculated by multiplication by "3" (the number of repeats of the test). *: The control group and the edoxaban-alone group were compared by the paired t-test (Bonferroni correction). The adjusted P value was calculated by multiplication by "2" (the number of repeats of the test). | | | |

**[Table 3]**

| No. | Comparison group | Method of analysis | *P* value |
|---|---|---|---|
| 1 | Edoxaban 75 ng/mL + PCI 0.3 µg/mL vs PCI 0.3 µg/mL | Paired t-test (Bonferroni correction)* | <0.001 |
| | Edoxaban 75 ng/mL + PCI 0.3 µg/mL vs Edoxaban 75 ng/mL | | <0.001 |
| 2 | Edoxaban 75 ng/mL + PCI 1 µg/mL vs PCI 1 µg/mL | Paired t-test (Bonferroni correction)* | <0.001 |
| | Edoxaban 75 ng/mL + PCI 1 µg/mL vs Edoxaban 75 ng/mL | | <0.001 |
| 3 | Edoxaban 75 ng/mL + PCI 3 µg/mL vs PCI 3 µg/mL | Paired t-test (Bonferroni correction)* | 0.004 |
| | Edoxaban 75 ng/mL + PCI 3 µg/mL vs Edoxaban 75 ng/mL | | <0.001 |
| 4 | Edoxaban 150 ng/mL + PCI 0.3 µg/mL vs PCI 0.3 µg/mL | Paired t-test (Bonferroni correction)* | <0.001 |
| | Edoxaban 150 ng/mL + PCI 0.3 µg/mL vs Edoxaban 150 ng/mL | | 0.016 |
| 5 | Edoxaban 150 ng/mL + PCI 1 µg/mL vs PCI 1 µg/mL | Paired t-test (Bonferroni correction)* | 0.001 |
| | Edoxaban 150 ng/mL + PCI 1 µg/mL vs Edoxaban 150 ng/mL | | <0.001 |
| 6 | Edoxaban 150 ng/mL + PCI 3 µg/mL vs PCI 3 µg/mL | Paired t-test (Bonferroni correction)* | <0.001 |
| | Edoxaban 150 ng/mL + PCI 3 µg/mL vs Edoxaban 150 ng/mL | | 0.001 |
| 7 | Edoxaban 300 ng/mL + PCI 0.3 µg/mL vs PCI 0.3 µg/mL | Paired t-test (Bonferroni correction)* | <0.001 |
| | Edoxaban 300 ng/mL + PCI 0.3 µg/mL vs Edoxaban 150 ng/mL | | 0.267 |
| 8 | Edoxaban 300 ng/mL + PCI 1 µg/mL vs PCI 1 µg/mL | Paired t-test (Bonferroni correction)* | <0.001 |
| | Edoxaban 300 ng/mL + PCI 1 µg/mL vs Edoxaban 300 ng/mL | | 0.018 |

| | | | |
|---|---|---|---|
| *: The edoxaban-alone group or the PCI-alone group and the edoxaban + PCI combined use group were compared by the paired t-test (Bonferroni correction). The adjusted P value was calculated by multiplication by "2" (the number of repeats of the test). | | | |

**[Table 4]**

| No. | Comparison group | Method of analysis | *P* value |
|---|---|---|---|
| 9 | Edoxaban 300 ng/mL + PCI 3 µg/mL vs PCI 3 µg/mL | Paired t-test (Bonferroni correction)* | 0.003 |
| | Edoxaban 300 ng/mL + PCI 3 µg/mL vs Edoxaban 300 ng/mL | | 0.027 |
| 10 | Control vs PCI 0.3 µg/mL | Paired t-test (Bonferroni correction)# | <0.001 |
| | Control vs PCI 1 µg/mL | | <0.001 |
| | Control vs PCI 3 µg/mL | | <0.001 |
| 11 | Control vs Edoxaban 75 ng/mL | Paired t-test (Bonferroni correction)# | <0.001 |
| | Control vs Edoxaban 150 ng/mL | | <0.001 |
| | Control vs Edoxaban 300 ng/mL | | <0.001 |
| 12 | Control, PCI 0.3 µg/mL, PCI 1 µg/mL, PCI 3 µg/mL | Spearman's rank correlation coefficient hypothesis test | <0.001 |
| 13 | Control, Edoxaban 75 ng/mL, Edoxaban 150 ng/mL, Edoxaban 300 ng/mL | Spearman's rank correlation coefficient hypothesis test | <0.001 |
| 14 | PCI 0.3 µg/mL, PCI 0.3 µg/mL + Edoxaban 75 ng/mL, PCI 0.3 µg/mL + Edoxaban 150 ng/mL, PCI 0.3 µg/mL + Edoxaban 300 ng/mL | Spearman's rank correlation coefficient hypothesis test | <0.001 |
| 15 | PCI 1 µg/mL, PCI 1 µg/mL + Edoxaban 75 ng/mL, PCI 1 µg/mL + Edoxaban 150 ng/mL, PCI 1 µg/mL + Edoxaban 300 ng/mL | Spearman's rank correlation coefficient hypothesis test | <0.001 |
| 16 | PCI 3 µg/mL, PCI 3 µg/mL + Edoxaban 75 ng/mL, PCI 3 µg/mL + Edoxaban 150 ng/mL, PCI 3 µg/mL + Edoxaban 300 ng/mL | Spearman's rank correlation coefficient hypothesis test | <0.001 |

| | | | |
|---|---|---|---|
| *: The edoxaban-alone group or the PCI-alone group and the edoxaban + PCI combined use group were compared by the paired t-test (Bonferroni correction). The adjusted P value was calculated by multiplication by "2" (the number of repeats of the test). #: The control group and the edoxaban-alone group or the PCI-alone group were compared by the paired t-test (Bonferroni correction). The adjusted P value was calculated by multiplication by "3" (the number of repeats of the test). | | | |

## Claims

1. A pharmaceutical composition for the promotion of fibrinolysis, comprising edoxaban or a pharmaceutically acceptable salt thereof, or a hydrate of the compound or the salt.

2. The pharmaceutical composition according to claim 1, further comprising a TAFIa inhibitor.

3. The pharmaceutical composition according to claim 2, wherein the TAFIa inhibitor is a carboxypeptidase inhibitor from potato tuber.

4. Use of edoxaban or a pharmaceutically acceptable salt thereof, or a hydrate of the compound or the salt for producing a pharmaceutical composition for the promotion of fibrinolysis.

5. The use according to claim 4, wherein a TAFIa inhibitor is further used.

6. The use according to claim 5, wherein the TAFIa inhibitor is a carboxypeptidase inhibitor from potato tuber.

7. A method for promoting fibrinolysis, comprising administering edoxaban or a pharmaceutically acceptable salt thereof, or a hydrate of the compound or the salt to a mammal.

8. The method according to claim 7, comprising administering a TAFIa inhibitor to the mammal at the same time as or separately from the edoxaban or the pharmaceutically acceptable salt thereof, or the hydrate of the compound or the salt.

9. The method according to claim 7 or 8, wherein the mammal is a human.

10. An agent promoting fibrinolysis, comprising edoxaban or a pharmaceutically acceptable salt thereof, or a hydrate of the compound or the salt in combination with a TAFIa inhibitor.
